(19) Europäisches Patentamt / European Patent Office / Office européen des brevets

(11) **EP 2 440 915 B1**

(12) **FASCICULE DE BREVET EUROPEEN**

(45) Date de publication et mention
de la délivrance du brevet:
**13.08.2014 Bulletin 2014/33**

(51) Int Cl.:
***B01D 46/00*** *(2006.01)*

(21) Numéro de dépôt: **10725152.2**

(86) Numéro de dépôt international:
**PCT/EP2010/058251**

(22) Date de dépôt: **11.06.2010**

(87) Numéro de publication internationale:
**WO 2010/142792 (16.12.2010 Gazette 2010/50)**

(54) **DISPOSITIF ET PROCEDE D'ANALYSE DE GAZ**

VORRICHTUNG UND VERFAHREN ZUR GASANALYSE

DEVICE AND METHOD FOR GAS ANALYSIS

(84) Etats contractants désignés:
**AL AT BE BG CH CY CZ DE DK EE ES FI FR GB
GR HR HU IE IS IT LI LT LU LV MC MK MT NL NO
PL PT RO SE SI SK SM TR**

(30) Priorité: **12.06.2009 FR 0902850**

(43) Date de publication de la demande:
**18.04.2012 Bulletin 2012/16**

(73) Titulaire: **Adixen Vacuum Products
74000 Annecy (FR)**

(72) Inventeurs:
 • **FAVRE, Arnaud
  F-74009 Annecy (FR)**
 • **GODOT, Erwan
  F-74009 Annecy (FR)**
 • **BELLET, Bertrand
  F-73000 Chambéry (FR)**

(74) Mandataire: **Croonenbroek, Thomas Jakob et al
Innovincia
11, avenue des Tilleuls
74200 Thonon-les-Bains (FR)**

(56) Documents cités:
 **EP-A1- 1 806 169     EP-A2- 0 684 470
 EP-A2- 1 703 547     US-A- 5 487 312**

 • **KONDOH EIICHI ET AL: "Measurements of trace
 gaseous ambient impurities on an atmospheric
 pressure rapid thermal processor" JOURNAL OF
 VACUUM SCIENCE AND TECHNOLOGY: PART A,
 AVS /AIP, MELVILLE, NY., US LNKD- DOI:
 10.1116/1.581632, vol. 17, no. 2, 1 mars 1999
 (1999-03-01), pages 650-656, XP012004429 ISSN:
 0734-2101**

## Description

La présente invention concerne un dispositif et un procédé d'analyse de gaz. L'invention concerne encore une station de mesure associée.

Les dispositifs d'analyse de gaz connus prélèvent un flux déterminé du gaz à analyser, par exemple à la sortie d'une enceinte de transport pour le convoyage et le stockage atmosphérique de substrats semi-conducteurs ou encore à l'entrée ou à la sortie d'un filtre (voir, par exemple, EP1806169) pour détecter la présence de traces de gaz de l'ordre du « ppb » pour "parts per billion" en anglais (partie par milliard en français).

De manière connue, un dispositif d'analyse comporte une pompe de prélèvement pour véhiculer un flux de gaz prélevé à une unité d'analyse ou analyseur.

Ces dispositifs d'analyse possèdent une gamme d'utilisation et une incertitude fixées par la technologie d'analyse ainsi que des moyens de calibration utilisés. En outre, ces dispositifs d'analyse ne fonctionnent correctement que pour des plages respectives de concentrations du gaz à analyser. Ainsi, chaque dispositif d'analyse présente une plage d'utilisation optimale en fonction de la nature du gaz à analyser.

Ceci présente l'inconvénient de nécessiter plusieurs dispositifs d'analyse en fonction des plages de mesure désirées. De cet inconvénient découlent des problèmes de coût et d'encombrement. Un moyen d'atténuer cet inconvénient est d'utiliser une unité de dilution en communication avec le dispositif d'analyse tel que décrit dans EP0684470. Cela implique de plus de connaître très précisément la plage de concentration des gaz à analyser afin d'optimiser le choix du dispositif d'analyse.

Par ailleurs, pour une plage de concentrations donnée, les dispositifs d'analyse peuvent généralement analyser par multiplexage différents flux de gaz par l'intermédiaire d'une ou plusieurs vannes multivoies. Par exemple, le dispositif d'analyse peut réaliser l'analyse d'un premier et d'un second flux de gaz, le premier flux de gaz étant de plus forte concentration que le second.

Cependant, si l'unité d'analyse reçoit d'abord le premier flux de gaz de plus forte concentration, l'unité d'analyse et la pompe de prélèvement peuvent être polluées, par exemple à cause de colmatage dans la pompe ou du dégazage de composés accumulés dans la pompe qui viennent polluer le flux de gaz à analyser. Cette pollution peut engendrer une diminution de la qualité de l'analyse.

De plus, un temps de réponse relativement long peut être nécessaire pour éliminer les gaz résiduels avant de réaliser l'analyse du deuxième flux de gaz.

L'invention a donc pour but de proposer un dispositif d'analyse de gaz aux performances optimisées avec une plage d'utilisation étendue, un temps de réponse diminué, et dont les problèmes de pollution dûs aux analyses de gaz de différentes plages de concentration sont réduits.

À cet effet, l'invention a pour objet un dispositif d'analyse de gaz pour déterminer la concentration du gaz à analyser, ledit dispositif d'analyse comprenant :

- une unité de dilution configurée pour diluer un flux de gaz à analyser selon un coefficient de dilution, et
- une unité d'analyse communiquant avec l'unité de dilution via une canalisation de prélèvement pour prélever par pompage un flux de gaz dilué, et comportant au moins un moyen de traitement pour :

  • analyser le flux de gaz dilué prélevé, et
  • déterminer la concentration du flux de gaz à analyser à partir dudit flux de gaz dilué analysé et du coefficient de dilution.

En outre, le dispositif d'analyse de gaz selon l'invention est configuré pour analyser d'une part un flux de gaz en entrée d'un filtre et d'autre part un flux de gaz en sortie du filtre.

En faisant varier la dilution du flux de gaz à analyser, on maintient au niveau de l'unité d'analyse un flux constant et des concentrations relativement faibles, ce qui permet d'utiliser une même unité d'analyse pour plusieurs gaz de plages de concentrations différentes.

De plus, on constate une amélioration du temps de réponse de l'unité, puisqu'un moindre temps d'attente est nécessaire pour éliminer le gaz résiduel dans la canalisation de prélèvement par rapport à un dispositif analysant un flux de gaz pur non dilué.

Un tel dispositif d'analyse permet en outre de réduire les risques de contamination de l'unité d'analyse et de la canalisation de prélèvement car les flux de gaz les traversant sont toujours dilués.

Le dispositif d'analyse de gaz selon l'invention peut en outre comporter une ou plusieurs caractéristiques suivantes, prises séparément ou en combinaison :

- l'unité de dilution est montée en dérivation par rapport à ladite canalisation,
- l'unité de dilution comporte une pluralité de voies de dilution montées en dérivation par rapport à ladite canalisation, chaque voie de dilution étant respectivement associée à un coefficient de dilution,
- chaque voie de dilution présente :

  • un moyen d'injection d'un flux de gaz neutre dans ladite canalisation pour diluer ledit flux de gaz à analyser, et
  • un moyen de pompage d'un flux de gaz dilué pour l'extraire de ladite canalisation, de manière à maintenir un flux constant dans ladite canalisation,

- chaque voie de dilution est respectivement associée à la dilution d'un flux de gaz à analyser,
- au moins deux voies de dilution sont associées pour la dilution d'un flux de gaz à analyser,

L'invention concerne encore un procédé d'analyse de gaz pour déterminer la concentration du gaz à analyser au moyen d'un dispositif d'analyse de gaz selon l'invention, comprenant les étapes suivantes :

- on détermine un coefficient de dilution,
- on injecte un flux de gaz neutre prédéterminé et on pompe un flux de gaz dilué, de manière à diluer un flux de gaz à analyser,
- on prélève par pompage un flux de gaz dilué,
- on analyse le flux de gaz dilué d'une part en entrée d'un filtre et d'autre part en sortie du filtre,
- on détermine la concentration du flux de gaz à analyser à partir dudit flux de gaz dilué analysé et du coefficient de dilution.

Ledit procédé d'analyse peut comprendre une étape préliminaire dans laquelle on détermine le coefficient de dilution en fonction de la valeur de concentration maximale de la plage de concentrations du gaz à analyser.

D'autres caractéristiques et avantages de l'invention ressortiront de la description suivante, donnée à titre d'exemple, sans caractère limitatif, en regard des dessins annexés sur lesquels :

- la figure 1 représente un dispositif d'analyse de gaz selon un premier mode de réalisation,
- la figure 2 représente un dispositif d'analyse de gaz selon un deuxième mode de réalisation,
- la figure 3 représente un dispositif d'analyse de gaz selon un troisième mode de réalisation, et
- la figure 4 illustre les différentes étapes d'un procédé d'analyse de gaz.

Sur ces figures, les éléments sensiblement identiques portent les mêmes références.

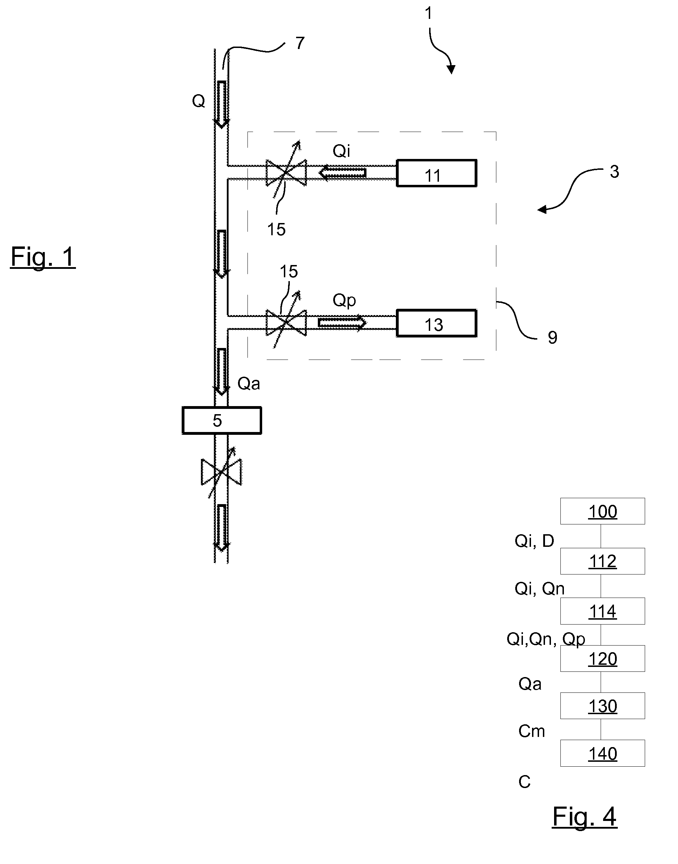

La figure 1 représente un dispositif d'analyse de gaz 1 pour déterminer la concentration du gaz à analyser, par exemple du gaz ammoniaque ayant une concentration de l'ordre de 5000 ppb. Plus précisément, dans un mélange gazeux, un tel dispositif d'analyse 1 peut déterminer la concentration en un gaz donné à partir d'un flux de gaz Q, même en de faibles proportions. La valeur du flux de gaz Q est vérifié par la relation (1).

$$(1)\ Q = S * P,\ \text{(avec Q=flux de gaz, S=vitesse de pompage, P=pression)}$$

À titre d'exemple, le dispositif d'analyse 1 comporte une unité d'analyse 5 ayant une plage d'utilisation de 0 à 50 ppb. L'analyse du gaz ammoniaque par exemple nécessite donc de diluer d'au moins cent fois le gaz.

Pour cela, le dispositif d'analyse 1 comporte une unité de dilution 3 du flux de gaz à analyser Q selon un coefficient de dilution D communiquant avec l'unité d'analyse 5 via une canalisation de prélèvement 7.

Pour prélever un flux de gaz dilué pour analyse Qa, on prévoit une pompe non représentée, qui peut être soit reliée à la canalisation 7, intégrée à l'unité d'analyse 5 ou encore disposée en amont ou aval de l'unité d'analyse 5. Le flux de gaz dilué prélevé pour analyse Qa est imposé par l'unité d'analyse 5.

L'unité d'analyse 5 comporte au moins un moyen de traitement pour :

- analyser le flux de gaz dilué prélevé Qa, et
- déterminer la concentration du flux de gaz à analyser Q à partir du flux de gaz dilué analysé Qa et du coefficient de dilution D.

Par exemple, l'unité d'analyse 5 comporte un analyseur de gaz (non représenté) pour mesurer la concentration Cm (figure 4) du flux de gaz dilué prélevé Qa.

Pour réaliser une analyse en temps réel, c'est-à-dire dans un laps de temps très court et de manière suffisamment sensible pour détecter de très faibles niveaux de contamination gazeuse à l'état de trace (de l'ordre du ppb), une possibilité est d'utiliser un analyseur de gaz dans lequel on mesure la mobilité des ions par exemple selon le principe de l'instrumentation IMS (« Ion Mobility Spectrometer » en anglais) ou selon la technologie IAMS (« Ion Attachment Mass Spectrometer » en anglais).

En outre, l'unité d'analyse 5 comporte un moyen de calcul (non représenté) de la concentration C du flux de gaz à analyser Q en multipliant la concentration mesurée Cm du flux de gaz dilué prélevé Qa par le coefficient de dilution D.

Ce coefficient de dilution D est déterminé de sorte que le flux de gaz dilué prélevé Qa analysé par l'unité d'analyse 5 corresponde à la plage d'utilisation de l'unité d'analyse 5. Pour cela, on détermine la valeur de concentration maximale de la plage de concentration du gaz à analyser que l'on peut avoir et on fixe le coefficient de dilution D en fonction de cette valeur.

On comprend donc que le coefficient de dilution D peut être adapté pour chaque plage de concentration du gaz à analyser. Ainsi, pour plusieurs gaz à analyser avec des plages de concentrations différentes, la dilution varie de manière à ce qu'une seule et même unité d'analyse puisse être utilisée.

De plus, dans la pompe de l'unité d'analyse 5 et dans l'unité d'analyse 5 passent uniquement des flux de gaz dilués, ce qui réduit le risque d'altérer la qualité de mesure de l'unité d'analyse. La dilution permet en outre d'éviter une contamination critique de l'unité d'analyse 5 car on maintient des valeurs de concentrations relativement faibles dans cette unité d'analyse 5.

En se référant aux figures 1 à 3, l'unité de dilution 3 peut présenter une ou plusieurs voies de dilution 9 montées en dérivation par rapport à la canalisation 7. Ces voies de dilution 9 sont encadrées en pointillés sur les figures 1 à 3.

Dans l'exemple illustré sur la figure 1, l'unité de dilution 3 comporte une seule voie de dilution 9. Cette voie de dilution 9 comporte deux branches en dérivation par rapport à la canalisation 7.

La première branche présente un moyen d'injection 11 d'un flux de gaz neutre Qi dans la canalisation 7 pour diluer le flux de gaz à analyser Q. On entend ici par « gaz neutre » un gaz inerte tel que de l'azote. La valeur du flux de gaz neutre injecté Qi est limitée par le flux disponible de l'installation où le dispositif est mis en place. On choisit la valeur du flux de gaz neutre Qi judicieusement la plus grande possible en prenant en compte le coût d'exploitation et la taille de l'installation. Par ailleurs, si on injecte trop de flux proche de l'analyseur on perturbe l'analyseur.

La deuxième branche présente un moyen de pompage 13 qui permet d'aspirer le flux de gaz à analyser Q dans la canalisation 7. La valeur du flux de gaz à analyser Q est déduite à partir du coefficient de dilution D et du flux de gaz neutre Qi selon la relation (2).

$$(2) \quad Q = \frac{Qi}{(D-1)}$$

Le moyen de pompage 13 permet d'autre part d'extraire un flux de gaz dilué Qp de la canalisation 7, de manière à maintenir un flux constant dans la canalisation 7.

Le flux de gaz dilué pompé Qp est alors calculé à l'aide de la relation (3).

$$(3) \quad Q = - (Qi + Qp + Qa)$$

A titre d'exemple, pour un flux de gaz neutre injecté Qi de 4.5 slm (slm="Standard Liter per Minute" en anglais, soit le débit en L.min$^{-1}$, 1 slm = 1.6883 Pa.m$^3$.s$^{-1}$), pour un flux de gaz dilué pompé Qp de -4.5 slm et pour un flux de gaz prélevé pour analyse Qa de 0.5 slm, le flux de gaz à analyser Q est égal à 0.5 slm selon la relation (3).

Le coefficient de dilution D est quant à lui égal à 10 selon la relation (2).

Le dispositif d'analyse 1 peut en outre comporter des débitmètres 15 permettant de réguler respectivement le flux de gaz neutre injecté Qi, le flux de gaz dilué pompé Qp, et le flux de gaz dilué prélevé pour analyse Qa.

On peut prévoir un pilotage automatique de ces débitmètres 15 par des moyens de contrôle (non représentés) pour contrôler et faire varier ces différents flux.

En variante, ces flux peuvent être déterminés par des micro-fuites.

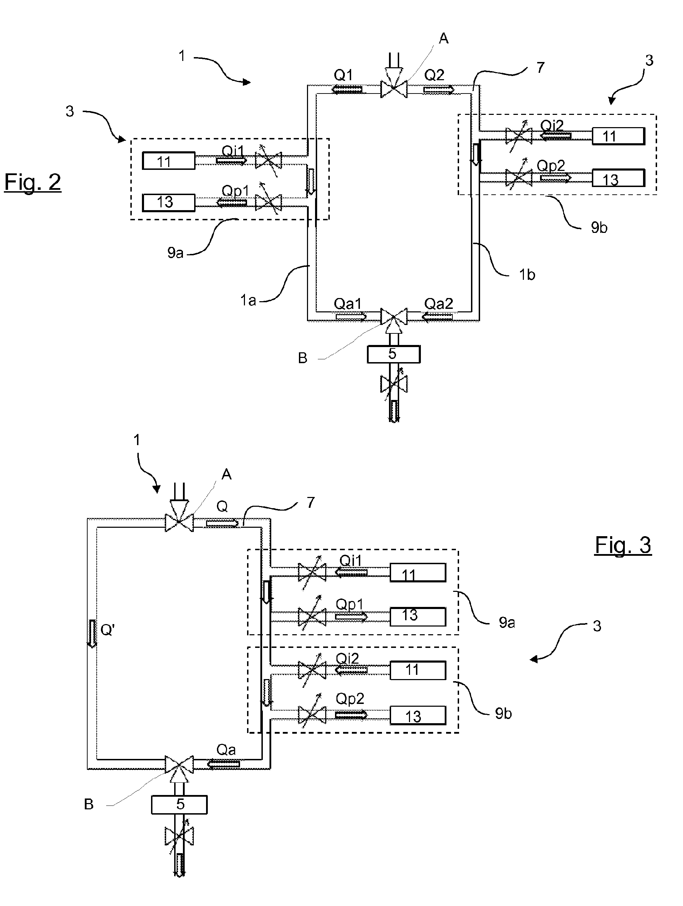

La figure 2 illustre un deuxième mode de réalisation dans lequel l'unité de dilution 3 comporte une première voie de dilution 9a sur une première branche 1a du dispositif d'analyse 1, et une seconde voie de dilution 9b sur une seconde branche 1b du dispositif d'analyse 1, les deux voies de dilution 9a et 9b étant montées en dérivation par rapport à la canalisation 7.

Comme on le remarque sur la figure 2, les deux branches 1a et 1b sont montées en parallèle, partent d'un point A commun pour l'introduction du flux de gaz à analyser Q1 ou Q2, et se rejoignent en un point B commun à l'entrée de l'unité d'analyse 5.

A cet effet, le dispositif d'analyse 1 comporte :

- des premières vannes multivoies 17a pour orienter le flux de gaz à analyser Q1 ou Q2 dans la branche correspondante selon l'analyse à effectuer, et
- des secondes vannes multivoies 17b pour permettre de prélever le flux de gaz dilué à analyser Qa1 ou Qa2 de la première 1a ou de la seconde 1b branche, selon l'analyse à effectuer.

Chaque voie de dilution 9a,9b peut être respectivement configurée pour diluer un flux de gaz à analyser associé selon un premier D1 et un deuxième D2 coefficients de dilution associés. Dans ce cas, pour chaque gaz à analyser, on utilise la voie de dilution associée 9a ou 9b et on utilise la même unité d'analyse 5. Pour déterminer la concentration du flux de gaz à analyser Q, on prend alors en compte le coefficient de dilution D1 ou D2 associé à la voie de dilution 9a ou 9b utilisée.

À titre d'exemple, l'unité d'analyse 5 impose un flux dilué à analyser Qa=0.3 slm.

Lorsqu'on associe la première voie de dilution 9a à une première plage de concentration de gaz à analyser, on détermine un premier coefficient de dilution D1 à partir de la concentration maximale de cette plage de concentration, par exemple D1=10.

Pour la dilution, le flux de gaz neutre à injecter Qi1 est imposé, par exemple Qi1=2.7 slm.

On déduit à partir du premier coefficient de dilution D1 et du flux de gaz neutre Qi1 (relation (2)), la valeur du flux Q1, dans cet exemple Q1=0.3 slm.

Puis on calcule (relation (3)), la valeur du flux dilué à pomper Qp1 pour diluer le flux de gaz Q1, dans cet exemple Qp1=-2.7 slm.

Une fois le flux de gaz dilué prélevé Qa1 analysé, on détermine la concentration du flux de gaz à analyser Q1 à partir du premier coefficient de dilution D1.

De même, lorsque la deuxième voie de dilution 9b est associée à une deuxième plage de concentration, on détermine un deuxième coefficient de dilution D2 à partir de la concentration maximale de cette plage de concentration, par exemple D2=20.

Pour la dilution, le flux de gaz neutre à injecter Qi2 est imposé, par exemple Qi2=5.4 slm.

On déduit à partir du deuxième coefficient de dilution D2 et du flux de gaz neutre Qi2 (relation (2)), la valeur du flux Q2, dans cet exemple Q2=0.3 slm.

Puis on calcule (relation (3)), la valeur du flux dilué à pomper Qp2 pour diluer le flux de gaz Q2, dans cet exemple Qp2=-5.4 slm.

Une fois le flux de gaz dilué prélevé Qa2 analysé, on détermine la concentration du flux de gaz à analyser Q2 à partir du deuxième coefficient de dilution D2.

En variante, on peut prévoir que pour chaque gaz à analyser, plus précisément pour chaque plage de concentration, on associe une ou plusieurs voies de dilution.

Selon un troisième mode de réalisation illustré sur la figure 3, on peut associer l'ensemble des voies de dilution en dérivation par rapport à la canalisation 7, par exemple la première 9a et la deuxième 9b voies de dilution, pour une plage de concentration de gaz donnée. Dans ce cas, les voies de dilutions sont montées en série sur une même branche, ici la branche 1b, du dispositif d'analyse 1.

En outre, le dispositif d'analyse 1 comporte une branche de contournement 1a sans voies de dilution pour la circulation du flux de gaz à analyser Q lorsque ce dernier ne doit pas être dilué.

Par exemple, si la plage de concentration du gaz n'est pas connue, on associe dès le départ les deux voies de dilution 9a et 9b, et si la concentration Cm du flux de gaz dilué prélevé Qa ne peut pas être déterminée car elle est trop faible, le flux de gaz Q est alors orienté sur la branche de contournement ne comportant pas de voie de dilution.

Dans ce cas, les premières vannes 17a permettent de distribuer le flux de gaz Q ou Q' dans la branche correspondante 1a ou 1b, et les secondes vannes 17b permettent de prélever le flux Q' ou le flux dilué Qa pour analyse.

Lorsque plusieurs voies de dilution sont utilisées en série, on détermine un coefficient de dilution global D égal au produit des coefficients de dilution de chaque voie de dilution utilisée, selon la relation (3).

$$D = \prod_i Di$$

(4)

Par exemple, on peut prévoir que pour une troisième plage de concentration de gaz à analyser, on associe les deux voies de dilution 9a et 9b pour diluer le flux de gaz à analyser Q. Dans ce cas, selon la relation (4), le coefficient global de dilution D est le produit des premier D1 et deuxième D2 coefficients de dilution.

De plus, selon ce troisième mode de réalisation les différents coefficients de dilution sont égaux (relation 5).

$$(5)\ Di = Dj\ (\text{pour tout } i,j)$$

Par conséquent, on peut déterminer la valeur d'un coefficient de dilution Di à partir du coefficient de dilution global D, selon la relation (6).

$$(6)\ Di = \sqrt[n]{D}\ (n = \text{nombre de voies de dilution})$$

Ainsi selon les relations (5) et (6), dans l'exemple illustré sur la figure 3, D1=D2=√D.

Un tel dispositif d'analyse selon l'invention est donc configuré pour analyser d'une part un flux de gaz en entrée d'un filtre et d'autre part un flux de gaz en sortie du filtre. En effet, bien que les concentrations de gaz diffèrent en entrée et en sortie du filtre, la concentration en sortie étant largement inférieure à la concentration en entrée le même dispositif d'analyse peut assurer les deux mesures.

Dans un mode de réalisation en dehors du cadre de la présente invention, un tel dispositif d'analyse peut être configuré pour analyser le gaz contenu dans une enceinte de transport pour le convoyage et le stockage atmosphérique de substrats semi-conducteurs. Le dispositif d'analyse peut par exemple faire partie d'une station de mesure de la contamination de l'enceinte qui s'accouple avec une telle enceinte pour la mesure.

En effet, dans les procédés de fabrication de semi-conducteurs ou de microsystèmes électromécaniques (MEMS), les substrats tels que les plaquettes (« wafers ») et les masques sont habituellement transportés et/ou stockés entre les étapes de procédé dans une enceinte de transport et/ou de stockage standardisée à ouverture latérale de type FOUP (« Front Opening Unified Pod ») ou à ouverture par le fond de type SMIF (« Standard Mechanical Actionneur »).

Ces enceintes de transport et/ou de stockage et leur atmosphère intérieure sont à pression atmosphérique d'air ou d'azote.

Les gaz contenus dans l'enceinte peuvent être analysés par une station de mesure placée en salle blanche par exemple pour former un poste de contrôle, ou encore par une chambre d'entrée/sortie d'équipement de fabrication semi-conducteur, comprenant un dispositif d'analyse pour le contrôle de la contamination gazeuse des substrats ou encore des enceintes elles-mêmes.

Ainsi, le dispositif d'analyse précédemment décrit et selon la présente invention met en oeuvre un procédé d'analyse de gaz pour déterminer la concentration du gaz à analyser (figure 4).

Ce procédé d'analyse peut comporter une étape préliminaire 100 dans laquelle on détermine le coefficient de dilution D d'une ou plusieurs voies de dilution, en fonction de la valeur de concentration maximale de la plage de concentration du flux de gaz à analyser Q. Le flux de gaz à analyser Q étant déterminé à partir de ce coefficient de dilution D et d'un flux de gaz neutre à injecter Qi (relation (2)).

Ensuite, lors d'une étape 110 on dilue un flux de gaz à analyser Q selon le coefficient de dilution D. Pour cela, à l'étape 112 on injecte le flux de gaz neutre Qi prédéterminé, et à l'étape 114 on pompe un flux de gaz dilué Qp pour maintenir une pression sensiblement constante. Le flux de gaz dilué pompé Qp étant calculé à partir de la relation (3).

Par la suite à l'étape 120, on prélève par pompage un flux de gaz dilué Qa imposé par l'unité d'analyse 5, puis on analyse le flux de gaz dilué prélevé Qa à l'étape 130, par exemple en mesurant la concentration Cm du flux de gaz dilué prélevé Qa, avant de déterminer à l'étape 140 la concentration C du flux de gaz à analyser Q à partir du flux de gaz dilué analysé Qa et du coefficient de dilution D, par exemple en multipliant la concentration mesurée Cm par le coefficient de dilution D.

On comprend donc qu'un tel dispositif d'analyse avec une unité de dilution permet d'analyser une pluralité de gaz ayant des plages de concentrations différentes. En outre, la dilution du gaz à analyser prévient des risques de contamination et diminue le temps de réponse de l'unité d'analyse.

**Revendications**

1. Dispositif d'analyse de gaz pour déterminer la concentration du gaz à analyser, ledit dispositif d'analyse comprenant : une unité de dilution (3) configurée pour diluer un flux de gaz à analyser (Q) selon un coefficient de dilution (D), et une unité d'analyse (5) communiquant avec l'unité de dilution (3) via une canalisation de prélèvement (7) pour prélever par pompage un flux de gaz dilué (Qa,), et comportant au moins un moyen de traitement pour analyser le flux de gaz dilué prélevé (Qa), et déterminer la concentration (C) du flux de gaz à analyser (Q) à partir dudit flux de gaz dilué analysé (Qa) et du coefficient de dilution (D), **caractérisé en ce que** ledit dispositif d'analyse de gaz est configuré pour analyser d'une part un flux de gaz en entrée d'un filtre et d'autre part un flux de gaz en sortie du filtre.

2. Dispositif d'analyse selon la revendication 1, dans lequel l'unité de dilution (3) est montée en dérivation par rapport à ladite canalisation (7).

3. Dispositif d'analyse selon l'une des revendications 1 ou 2, dans lequel l'unité de dilution (3) comporte une pluralité de voies de dilution (9a,9b) montées en dérivation par rapport à ladite canalisation (7), chaque voie de dilution (9a,9b) étant respectivement associée à un coefficient de dilution (D1,D2).

4. Dispositif d'analyse selon la revendication 3, dans lequel chaque voie de dilution présente : un moyen d'injection (11) d'un flux de gaz neutre (Qi) dans ladite canalisation (7) pour diluer ledit flux de gaz à analyser (Q), et un moyen de pompage (13) d'un flux de gaz dilué (Qp) pour l'extraire de ladite canalisation (7), de manière à maintenir un flux constant dans ladite canalisation (7).

5. Dispositif d'analyse selon l'une des revendications 3 ou 4, dans lequel chaque voie de dilution (9a, 9b) est respectivement associée à la dilution d'un flux de gaz à analyser.

6. Dispositif d'analyse selon l'une des revendications 3 à 5, dans lequel au moins deux voies de dilution (9a, 9b) sont associées pour la dilution d'un flux de gaz à analyser.

7. Procédé d'analyse de gaz pour déterminer la concentration du gaz à analyser au moyen d'un dispositif d'analyse de gaz selon l'une des revendications 1 à 6, comprenant les étapes suivantes :

- on détermine un coefficient de dilution (D),
- on injecte un flux de gaz neutre (Qi) prédéterminé et on pompe un flux de gaz dilué (Qp), de manière à diluer un flux de gaz à analyser (Q),
- on prélève par pompage un flux de gaz à analyser dilué (Qa),
- on analyse le flux de gaz dilué prélevé (Qa) d'une part en entrée d'un filtre et d'autre part en sortie du filtre,
- on détermine la concentration (C) du flux de gaz à analyser (Q) à partir dudit flux de gaz dilué analysé (Qa) et du coefficient de dilution (D).

8. Procédé d'analyse selon la revendication 7, comprenant une étape préliminaire dans laquelle on détermine le coefficient de dilution (D) en fonction de la valeur de concentration maximale de la plage de concentrations du gaz à analyser.

## Patentansprüche

1. Gasanalysevorrichtung zum Bestimmen der Konzentration eines zu analysierenden Gases, wobei die Analysevorrichtung Folgendes umfasst: eine Verdünnungseinheit (3), die konfiguriert ist, um einen Strom (Q) eines zu analysierenden Gases in Übereinstimmung mit einem Verdünnungskoeffizienten (D) zu verdünnen, und eine Analyseeinheit (5), die mit der Verdünnungseinheit (3) über eine Abgriffleitung (7) kommuniziert, um durch Pumpen einen verdünnten Gasstrom (Qa) abzugreifen, und wenigstens ein Verarbeitungsmittel zum Analysieren des abgegriffenen verdünnten Gasstroms (Qa) und zum Bestimmen der Konzentration (C) des Stroms (Q) des zu analysierenden Gases anhand des analysierten verdünnten Gasstroms (Qa) und des Verdünnungskoeffizienten (D) enthält, **dadurch gekennzeichnet, dass** die Gasanalysevorrichtung konfiguriert ist, einerseits einen Gasstrom am Eingang eines Filters und andererseits einen Gasstrom am Ausgang des Filters zu analysieren.

2. Analysevorrichtung nach Anspruch 1, wobei die Verdünnungseinheit (3) in einer Abzweigung in Bezug auf die Leitung (7) montiert ist.

3. Analysevorrichtung nach einem der Ansprüche 1 oder 2, wobei die Verdünnungseinheit (3) mehrere Verdünnungswege (9a, 9b) umfasst, die in einer Abzweigung in Bezug auf die Leitung (7) montiert sind, wobei jedem Verdünnungsweg (9a, 9b) ein entsprechender Verdünnungskoeffizient (D1, D2) zugeordnet ist.

4. Analysevorrichtung nach Anspruch 3, wobei jeder Verdünnungsweg aufweist: ein Mittel (11) zum Einspritzen eines Stroms (Qi) eines neutralen Gases in die Leitung (7), um den Strom (Q) des zu analysierenden Gases zu verdünnen, und ein Mittel (13) zum Pumpen eines Stroms (Qp) eines verdünnten Gases, um ihn aus der Leitung (7) zu extrahieren, derart, dass in der Leitung (7) ein konstanter Strom aufrechterhalten wird.

5. Analysevorrichtung nach einem der Ansprüche 3 oder 4, wobei jedem Verdünnungsweg (9a, 9b) jeweils eine Verdünnung eines Stroms des zu analysierenden Gases zugeordnet ist.

6. Analysevorrichtung nach einem der Ansprüche 3 bis 5, wobei wenigstens zwei Verdünnungswege (9a, 9b) der Verdünnung eines Stroms eines zu analysierenden Gases zugeordnet sind.

7. Gasanalyseverfahren zum Bestimmen der Konzentration eines zu analysierenden Gases mittels einer Gasanalysevorrichtung nach einem der Ansprüche 1 bis 6, das die folgenden Schritte umfasst:

- Bestimmen eines Verdünnungskoeffizienten (D),
- Einspritzen eines Stroms (Qi) eines vorgegebenen neutralen Gases und Pumpen eines Stroms (Qp) eines verdünnten Gases, derart, dass ein Strom (Q) eines zu analysierenden Gases verdünnt wird,
- Abgreifen durch Pumpen eines Stroms (Qa) eines verdünnten zu analysierenden Gases,
- Analysieren des abgegriffenen Stroms (Qa) eines verdünnten Gases einerseits am Eingang eines Filters und andererseits am Ausgang des Filters,
- Bestimmen der Konzentration (C) des Stroms (Q) des zu analysierenden Gases anhand des Stroms (Qa) des analysierten verdünnten Gases und des Verdünnungskoeffizienten (D).

8. Analyseverfahren nach Anspruch 7, das einen vorhergehenden Schritt umfasst, in dem der Verdünnungskoeffizient (D) als Funktion des Wertes der maximalen Konzentration des Bereichs von Konzentrationen des zu analysierenden Gases bestimmt wird.

**Claims**

1. Gas analysis device for determining the concentration of the gas to be analyzed, said analysis device comprising: a diluting unit (3) configured to dilute a flow of gas to be analyzed (Q) according to a dilution coefficient (D), and an analysis unit (5) communicating with the diluting unit (3) via a sampling pipe (7) in order to sample a flow of diluted gas (Qa) by pumping, and comprising at least one processing means for: analyzing the sampled flow of diluted gas (Qa), and determining the concentration (C) of the flow of gas to be analyzed (Q) from said analyzed flow of diluted gas (Qa) and from the dilution coefficient (D), **characterized in that** said gas analysis device is configured to analyse, on the one hand, a flow of gas at the input of a filter and, on the other hand, a flow of gas at the output of the filter.

2. Analysis device according to Claim 1, wherein the diluting unit (3) is connected as a branch with respect to said pipe (7).

3. Analysis device according to either of Claims 1 and 2, wherein the diluting unit (3) comprises a plurality of dilution channels (9a, 9b) connected as branches with respect to said pipe (7), each dilution channel (9a, 9b) being respectively associated with a dilution coefficient (Dl, D2).

4. Analysis device according to Claim 3, wherein each dilution channel has:

- a means of injection (11) of a flow of neutral gas (Qi) into said pipe (7) in order to dilute said flow of gas to be analyzed (Q), and
- a means of pumping (13) a flow of diluted gas (Qp) in order to extract it from said pipe (7), in such a way as to maintain a constant flow in said pipe (7).

5. Analysis device according to either of Claims 3 and 4, wherein each dilution channel (9a, 9b) is respectively associated with the dilution of a flow of gas to be analysed.

6. Analysis device according to one of Claims 3 to 5, wherein at least two dilution channels (9a, 9b) are associated for the dilution of a flow of gas to be analysed.

7. Gas analysis method for determining the concentration of the gas to be analysed by means of a gas analysis device according to one of Claims 1 to 6, comprising the following steps:

- a dilution coefficient (D) is determined,

- a predetermined flow of neutral gas (Qi) is injected and a flow of diluted gas (Qp) is pumped, in such a way as to dilute a flow of gas to be analyzed (Q),
- a flow of diluted gas to be analyzed (Qa) is sampled by pumping,
- the sampled flow of diluted gas (Qa) is analysed on the one hand at the input of a filter and on the other hand at the output of the filter,
- the concentration (C) of the flow of gas to be analyzed (Q) is determined from said analyzed flow of diluted gas (Qa) and from the dilution coefficient (D).

8.  Gas analysis method according to Claim 7, comprising a preliminary step in which the dilution coefficient (D) is determined as a function of the maximum value of concentration of the range of concentration of the gas to be analysed.

Fig. 1

Fig. 4

Fig. 2

Fig. 3

## RÉFÉRENCES CITÉES DANS LA DESCRIPTION

**Documents brevets cités dans la description**

- EP 1806169 A
- EP 0684470 A